# EUROPÄISCHE PATENTSCHRIFT

(11) **EP 0 720 856 B1**
(45) Veröffentlichungstag und Bekanntmachung des Hinweises auf die Patenterteilung: **17.10.2001**
(21) Anmeldenummer: 96103982.3
(22) Anmeldetag: 11.03.1993
(51) Int. Cl.: A61M 1/16

(54) **Anordnung zum On-Line Spülen und Befüllen eines extrakorporalen Blutkreislaufes von Dialysiermaschinen**
Device for on-line cleaning and filling of an extracorporal blood circuit of a dialysis apparatus
Dispositif de nettoyage et remplissage en ligne d'un circuit extracorporel sanguin d'une machine de dialyse

(30) Priorität: 13.03.1992 DE 4208274
(43) Veröffentlichungstag der Anmeldung: 10.07.1996
(62) Teilanmeldung aus: 93103965.5
(73) Patentinhaber: MEDICAL SUPPORT GMBH, D-63110 Rodgau (DE)
(72) Erfinder: Eigendorf, Hans-Günther, Dr., 15526 Bad Saarow-Pieskow (DE)
(74) Vertreter: Flosdorff, Jürgen, Dr.

(56) Entgegenhaltungen:
- EP-A- 0 199 518
- EP-A- 0 234 001
- DE-C- 3 936 785
- FR-A- 2 175 945
- FR-A- 2 296 432
- FR-A- 2 368 963
- US-A- 4 331 540
- US-A- 4 784 771

## Beschreibung

Die Erfindung betrifft eine Anordnung zum Spülen und Befüllen eines extrakorporalen Blutkreislaufs von Dialysemaschinen, insbesondere von Blutschlauchleitungen und Dialysatoren. Das Befüllen beinhaltet dabei die Entlüftung des extrakorporalen Blutkreislaufs, die für eine Dialysebehandlung unabdingbare Voraussetzung ist.

Als arterielle und venöse Blutschlauchleitung eines extrakorporalen Blutkreislaufs werden heutzutage üblicherweise steril verpackte Einmalartikel verwendet, die vor einer Behandlung gespült, entlüftet und befüllt werden müssen. Dies geschieht bisher mittels physiologischer Kochsalzlösungen, die mittels der maschineneigenen Blutpumpe durch das Schlauchsystem und den Dialysator gefördert werden, um den extrakorporalen Blutkreislauf von Verunreinigungen freizuspülen und unter vollständiger Entlüftung zu befüllen.

Die Verwendung der Kochsalzspülflüssigkeit hat den Nachteil, daß sie mit erheblichen Kosten verbunden ist.

In der FR-A-2 175 945 wird vorgeschlagen, die Dialysierflüssigkeit zum Spülen des extrakorporalen Blutkreislaufs nach Beendigung einer Dialysebehandlung zu verwenden. Hierbei saugt die Blutpumpe Dialysierflüssigkeit aus einem Dialysierflüssigkeitsbehälter in das Blutschlauchsystem ein.

Der vorliegenden Erfindung liegt die Aufgabe zugrunde, eine Anordnung anzugeben, mit der das Spülen, Entlüften und Befüllen eines extrakorporalen Blutkreislaufs von Dialysemaschinen vereinfacht ist.

Diese Aufgabe wird erfindungsgemäß durch die Merkmale des Anspruchs 1 gelöst. Vorteilhafte Weiterbildungen der Erfindung sind in den abhängigen Ansprüchen gekennzeichnet.

Gemäß der Erfindung wird die von der Dialysemaschine erzeugte bzw. aufbereitete Dialysierflüssigkeit zum Zwecke des Spülens, Entlüftens und Befüllens durch den bzw. in den extrakorporalen Blutkreislauf gefördert. Hierdurch entfällt das bisherige Erfordernis, eine in einem Beutel abgefüllte Kochsalzlösung zu diesem Zweck bereit zu stellen, der zudem von einer Bedienungsperson an die arterielle Blutschlauchleitung anzuschließen war. Es entfällt damit ferner das Erfordernis, zu dem genannten Zweck Kochsalzlösungen in größeren Mengen bereit zu halten und die geleerten Beutel zu entsorgen.

Gemäß der Erfindung wird die Dialysierflüssigkeit blutseitig des Dialysators abgenommen, wobei sie beim Durchtritt durch die Membran filtiert wird. Hiermit ist die Keimfreiheit der Spülund Befüllflüssigkeit zuverlässig gewährleistet.

Gemäß der Erfindung wird die Dialysierflüssigkeit zum Zwecke der Spülung, Entlüftung und Befüllung des extrakorporalen Blutkreislaufs von einer Pumpe des Dialysierflüssigkeitskreislaufs durch die bzw. in die Blutschlauchleitungen gepumpt. Hierbei kann dieser Vorgang durch das maschinenspezifische Füllprogramm hervorgerufen werden.

Erfindungsgemäß ist vorgesehen, daß die arterielle und die venöse Blutschlauchleitung in der Weise an dem Dialysator konnektiert werden, wie dies bei einer Dialysebehandlung der Fall ist. In dem Dialysierflüssigkeitskreislauf ist stromabwärts des Dialysators ein Dreiwege-Ventil mit 2/2 Wegen angeordnet, mit dem der Dialysierflüssigkeitskreislauf ganz oder teilweise verschließbar ist bzw. unterbrochen werden kann. Der dritte Anschluß dieses Dreiwege-Hahns, der vorzugsweise mechanisch einfach zu verstellen ist, liegt nicht im Dialysierflüssigkeitskreislauf und kann eine zur Atmosphäre hin offene Verbindung schaffen.

Zum Spülen und Befüllen des extrakorporlane Blutkreislaufs wird der Dreiwege-Hahn so geschaltet, daß der Dialysierflüssigkeitskreislauf zur Atmosphäre hin geöffnet ist, während die von dem Dialysator wegführende Leitung ganz oder teilweise verschlossen ist. Es wird betont, daß eine Zwischenstellung des Dreiwegehahns eingestellt werden kann, in der noch ein Teil der Dialysierflüssigkeit über die Ventilanordnung abfließen kann, was weiter unten noch erläutert wird.

Wenn der Dialysierflüssigkeitskreislauf durch entsprechende Schaltung des Dreiwegehahns zur Atmosphäre hin geöffnet ist, kann die maschineneigene Sensorik die Luft erkennen, die über den zugeordneten Anschluß ins Maschineninnere gelangt, und das maschinenspezifische Füllprogramm auslösen. Die dabei von der Maschine kommende Dialysierflüssigkeit wird über den Dialysator in die beiden angeschlossenen Blutschlauchleitungen gedrückt, da der Dreiwegehahn den Dialysierflüssigkeitskreislauf ganz oder teilweise unterbrochen hat. Somit werden die Blutschlauchleitungen gespült, entlüftet und mit der Dialysierflüssigkeit befüllt.

Die patientenseitigen Anschlüsse werden nach einem weiteren Vorschlag der Erfindung beispielsweise an einen leeren Beutel konnektiert. In diesem Fall kann es sich empfehlen, den Dreiwegehahn auf eine Zwischenstellung zu schalten, um den Dialysierflüssigkeitskreislauf nur teilweise zu verschließen, damit der Beutel nicht mit einer zu großen Spülflüssigkeitsmenge gefüllt bzw. nicht mit zu großem Druck belastet wird, der ihn platzen lassen könnte.

Nach einem alternativen Vorschlag der Erfindung können die freien Enden der Blutschlauchleitungen so angeordnet werden, daß sie in ein offenes Gefäß einmünden.

Bei dieser letztgenannten Ausführungsform der erfindungsgemäßen Anordnung wird das arterielle Pumpsegment nicht in die Blutpumpe eingelegt, da die Befüllung der arteriellen Blutschlauchleitung in umgekehrter Richtung erfolgt.

Ein Vorteil der Anordnung besteht darin, daß dialysatorseitig keine sterilen Einmalartikel benötigt werden und daß die arterielle und die venöse Blutschlauchleitung wie gewohnt konnektiert werden und unverändert bleiben.

Die Spül- und Befüllvorgang muß nicht notwendigerweise durch das maschinenspezifische Füllprogramm ausgelöst werden, welches auf ins Maschineninnere gelangte Luft anspricht.

Nach einem zusätzlichen Vorschlag der Erfindung ist vorgesehen, daß ein Sterilfilter stromaufwärts des Dialysators in dem Dialysierflüssigkeitskreislauf angeordnet wird, um die Sicherheit in Bezug auf die erforderliche Keimfreiheit zusätzlich zu erhöhen.

Nach einem weiteren Gesichtspunkt der Erfindung wird vorgeschlagen, daß die als Spülflüssigkeit verwendete Dialysierflüssigkeit nach Beendigung einer Dialysebehandlung zur Freispülung des extrakorporalen Blutkreislaufes verwendet wird, wozu es zweckmäßig ist, diese Flüssigkeit beim Spülvorgang in einen Beutel abzuführen. Das Freispülen ist notwendig, um nach der Dialysebehandlung das Blut vollständig zum Patienten zurückzuführen, was bisher ebenfalls mit Hilfe einer Kochsalzlösung geschieht.

Ein weiterer erfindungsgemäßer Vorschlag geht dahin, die extrakorporalen Komponenten so zu reinigen, daß sie wiederverwendbar sind. Bei einem dialysemaschinenspezifischen Reinigungsprogramm wird durch die Förderung der Blutpumpe Reinigungsflüssigkeit über die semipermeable Membran des Dialysators in den extrakorporalen Kreislauf gezogen, wodurch dieser entsprechend gereinigt wird.

Selbstverständlich liegt es im Rahmen der Erfindung, die extrakorporalen Komponenten weiterhin als Einmalartikel einzusetzen.

Weitere Merkmale, Vorteile und Einzelheiten der Erfindung ergeben sich aus der nachfolgenden Beschreibung einiger bevorzugter Ausführungsformen sowie anhand der Zeichnung. Dabei zeigen auf rein schematische Weise:
- Fig. 1: eine herkömmliche Anordnung;
- Fig. 2: eine Ausführungsform der erfindungsgemäßen Anordnung;

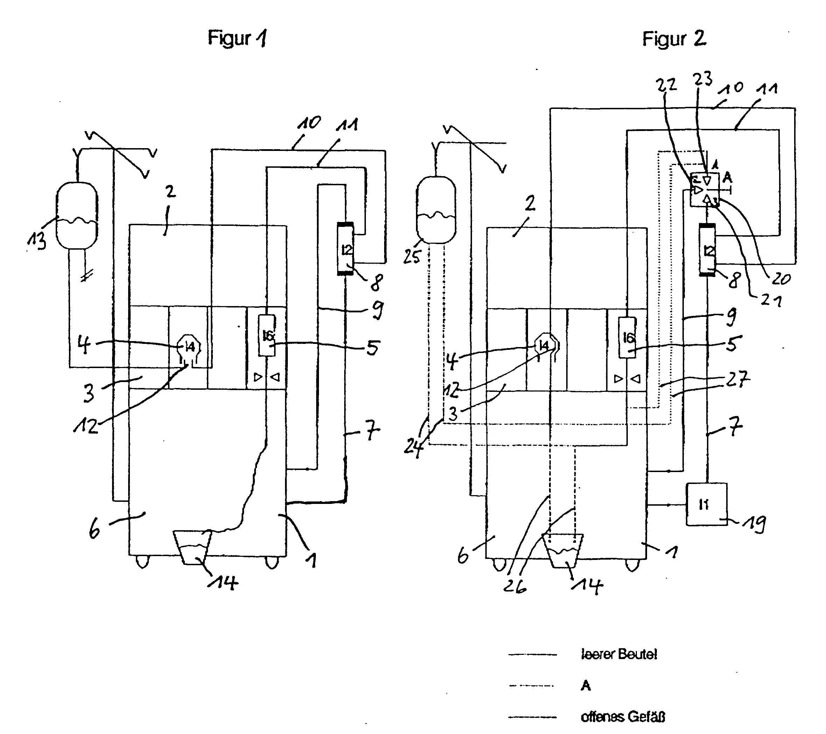

Es wird zunächst auf Fig. 1 Bezug genommen, um das bisher übliche Verfahren zum Spülen, Entlüften und Befüllen eines extrakorporalen Blutkreislaufs zu erläutern. Die Fig. zeigt eine eigentliche Dialysemaschine 1 mit der oberen überwachungsebene 2, der mittleren Ebene 3, die eine Blutpumpe 4 und einen Luftdetektor 5 enthält, und mit einer unteren Ebene 6, die den Hydraulikteil aufweist.

Die Dialysemaschine 1 ist über eine Zuleitung 7 mit dem Dialysator 8 verbunden, von dem eine Rückleitung 9 zur Dialysemaschine 1 zurückführt, um einen Dialysierflüssigkeitskreislauf zu schließen.

Zum Zwecke einer Dialysebehandlung werden eine arterielle Blutschlauchleitung 10 und eine venöse Blutschlauchleitung 11 an den dargestellten Stellen an dem Dialysator 8 konnektiert. Ein Blutpumpensegment 12 der arteriellen Blutschlauchleitung 10 wird in die Blutpumpe 4 eingelegt, während die venöse Blutschlauchleitung 11 über den Luftdetektor 5 führt.

Zur Vorbereitung einer Dialysebehandlung müssen die Blutschlauchleitungen 10 und 11 freigespült werden und der extrakorporale Blutkreislauf muß entlüftet und befüllt werden. Hierzu ist es bisher üblich, das freie Ende der arteriellen Blutschlauchleitung 10 mit dem Ausgang eines eine Kochsalzlösung enthaltenden Beutels 13 zu verbinden. Die Blutpumpe 4 fördert die Kochsalzlösung durch die arterielle Blutschlauchleitung 10, den Dialysator 8, die venöse Blutschlauchleitung 11 und den Luftdetektor 5, um schließlich die Spülflüssigkeit in einen offenen Behälter 14 abzuführen.

Es wird nun auf Fig. 2 Bezug genommen, in der die Bauteile, die mit denjenigen gemäß Fig. 1 übereinstimmen, mit denselben Bezugszeichen gekennzeichnet sind. Auf ihre Wiederholung wird aus Gründen der Übersichtlichkeit verzichtet.

Bei der erfindungsgemäßen Ausführungsform gemäß Fig. 2 werden die arterielle Blutschlauchleitung 10 und die venöse Blutschlauchleitung 11 in der Weise an dem Dialysator 8 konnektiert, wie dies im Zusammenhang mit der Figur 1 beschrieben und bei einer Dialysebehandlung der Fall ist. Bei dieser Ausführungsform ist ein Dreiwegehahn 20 in der Leitung 9 angeordnet, dessen Anschlüsse 21 und 22 der Leitung 9 zugeordnet sind, während sein Anschluß 23 normalerweise zur Atmosphäre hin offen ist.

Während einer Dialysebehandlung besteht eine Verbindung von 21 nach 22, während -wie gesagt- Anschluß 23 zur Atmosphäre hin offen ist. Zum Spülen des extrakorporalen Blutkreislaufs wird die Ventilanordnung 20 umgeschaltet auf Durchgang 22, 23, wobei auch eine Zwischenstellung schaltbar ist, in der ein eingeschränkter Durchgang 21, 22 bestehen bleibt. Von der Dialysemaschine 1 kommende Dialysierflüssigkeit wird über den Dialysator 8 in die Blutschlauchleitungen 10, 11 gedrückt, wobei diese durchgespült werden. Die Spülflüssigkeit kann entweder über Leitungen 24 in einen Beutel 25, über Leitungen 26 in das offene Gefäß 14 abgeführt oder über Leitungen 27 dem Anschluß 23 des Dreiwegeventils 20 zugeführt werden, von wo die Flüssigkeit in den Dialysierflüssigkeitskreislauf bzw. dessen Leitung 9 gelangt.

Bei dieser Ausführungsform ist das Blutpumpensegment nicht in die Blutpumpe 4 eingelegt.

In der Leitung 7 des Dialysierflüssigkeitskreislaufs ist ein Sterilfilter 19 angeordnet, der die Sicherheit der Keimfreiheit der Spülflüssigkeit weiter erhöht.

## Patentansprüche

1. Anordnung zum On-Line Spülen und Befüllen eines extrakorporalen Blutkreislaufs von Dialysemaschinen, insbesondere von Blutschlauchleitungen und Dialysatoren, wobei die arterielle (10) und die venöse (11) Blutschlauchleitung wie bei der Dialysebehandlung an dem Dialysator (8) konnektiert sind,
**dadurch gekennzeichnet,**
**daß** in dem Dialysierflüssigkeitskreislauf (7,8,9) stromabwärts des Dialysators ein Dreiwegeventil (20) angeordnet ist, mit dem die Rückleitung (9) des Dialysierflüssigkeitskreislaufs beim Spülen und Befüllen ganz oder teilweise verschließbar ist, wobei der dritte, nicht im Dialysierflüssigkeitskreislauf liegende Anschluß (23) des Dreiwegeventils (20) zur Atmosphäre hin offen ist.

2. Anordnung nach Anspruch 1,
**dadurch gekennzeichnet, daß** mit dem Dreiwegeventil (20) wenigstens eine Zwischenstellung einstellbar ist, bei der die Rückleitung (9) des Dialysierflüssigkeitskreislaufs teilweise geöffnet ist.

3. Anordnung nach Anspruch 1 oder 2,
**dadurch gekennzeichnet, daß** die patientenseitigen Anschlüsse der Blutschlauchleitungen (10, 11) an einem leeren Beutel (25) konnektiert sind.

4. Anordnung nach Anspruch 1 oder 2,
**dadurch gekennzeichnet, daß** die patientenseitigen Anschlüsse der Blutschlauchleitungen (10, 11) in einen offenen Behälter (14) einmünden.

5. Anordnung nach einem der Ansprüche 1 bis 4,
**dadurch gekennzeichnet, daß** ein Sterilfilter (19) in der Zuleitung (7) des Dialysierflüssigkeitskreislaufs stromaufwärts des Dialysators (8) angeordnet ist.

## Claims

1. Arrangement for the on-line flushing and filling of an extracorporeal blood circuit of dialysis machines, in particular of blood tube lines and dialysers, wherein the arterial (10) and the venous (11) blood tube line are connected to the dialyser (8) as in the case of dialysis treatment,
**characterised in**
**that** a three-way valve (20) is arranged in the dialysing fluid circuit (7, 8, 9) downstream of the dialyser, by means of which valve the return line (9) of the dialysing fluid circuit can be completely or partly closed during flushing and filling, wherein the third connection (23), which does not lie in the dialysing fluid circuit, of the three-way valve (20) is open to the atmosphere.

2. Arrangement according to Claim 1,
**characterised in that** at least one intermediate position can be set by means of the three-way valve (20), in which position the return line (9) of the dialysing fluid circuit is partly open.

3. Arrangement according to Claim 1 or 2,
**characterised in that** the connections of the blood tube lines (10, 11) on the patient side are connected to an empty bag (25).

4. Arrangement according to Claim 1 or 2,
**characterised in that** the connections of the blood tube lines (10, 11) on the patient side lead into an open container (14).

5. Arrangement according to any one of Claims 1 to 4,
**characterised in that** a sterile filter (19) is arranged in the feed line (7) of the dialysing fluid circuit upstream of the dialyser (8).

## Revendications

1. Agencement de nettoyage et de remplissage on ligne d'un circuit extracorporel sanguin de machines de dialyse, notamment de tuyaux souples de sang et de dialyseurs, où les tuyaux de sang artériels (10) et veineux (11), comme lors du traitement par dialyse, sont connectés au dialyseur (8), **caractérisé en ce qu'**il est disposé dans le circuit de liquide de dialyse (7, 8, 9) en aval du dialyseur une vanne à trois voies (20), par laquelle le tuyau de retour (9) du circuit de liquide de dialyse, lors du nettoyage et du remplissage, peut être fermé entièrement ou partiellement, où 1e troisième raccord (23) de la vanne à trois voies (20) qui ne se situe pas dans le circuit de liquide de dialyse est ouvert vers l'atmosphère.

2. Agencement selon la revendication 1, **caractérisé en ce qu'**au moyen de la vanne à trois voies (20), au moins une position intermédiaire peut être réglée à laquelle le tuyau de retour (9) du circuit de liquide de dialyse est partiellement ouvert.

3. Agencement selon la revendication 1 ou 2, **caractérisé en ce que** les raccords, côté patient, des tuyaux de sang (10, 11) sont connectés à un sac vide (25).

4. Agencement selon la revendication 1 ou 2, **caractérisé en ce que** les raccords, côté patient, des tuyaux de sang (10, 11) débouchent dans un récipient ouvert (14).

5. Agencement selon l'une des revendications 1 à 4, **caractérisé en ce qu'**un filtre stérile (19) est disposé dans le tuyau d'amenée (7) du circuit de liquide de dialyse en amont du dialyseur (8).
